# EUROPEAN PATENT APPLICATION

(11) **EP 1 736 925 A1**
(43) Date of publication of application: **27.12.2006**
(21) Application number: 05386016.9
(22) Date of filing: 16.06.2005
(51) Int. Cl.: G06Q 50/00, G07F 7/10

(54) **Electronic health book**

(30) Priority: 13.06.2005 GR 2005100293
(71) Applicant: Linax Holdings Ltd.,, Lefkosia (CY)
(72) Inventor: Liamis, Dimosthenis, 14561 Kifddias Attikis (GR)

(57) **Abstract**

The present device refers to the creation of a health book in the form of an electronic card of a dimension that is similar to that of the common credit card that provides information relating to the medical history and the medical insurance of the holder. The capacity for the reading and recording of particulars shall be provided through specific software, by the insurer and the connected doctors / hospitals, either locally (on the same computer), or through the Internet, thus abolishing the need for the production of the book by the insured or the compensation receipt clipping from the doctor to the offices of the insurer.
The book shall contain particulars in relation to the identity of the holder, personal medical information (blood group, allergies, etc), insurance particulars (coverage, authentications, etc) and the complete medical history with the capacity for the storage of text, images and electronic particulars relating to medical devices.
It provides the capacity to a specific category of users {Doctors at regional Hospitals - Rural Surgeries, etc.} to transmit medical information in relation to the patient, through the Internet to central Hospitals or University centres that remote medically support them. In this manner difficult emergency situations shall be successfully confronted.
In situations where the holder is not in possession of his faculties, the insertion of the detection system on the EHB makes its detection by the ambulance or Hospitals easy and quick. Furthermore the insertion of this system makes the detection of the EHB easy when its holder loses it or misplaces it. This is especially necessary for people with disabilities e.g. the blind.

## Description

The present device relates to the creation of a health book in the form of an electronic card of a dimension that is similar to that of the common credit card or of the dimension of a small flash-drive key (USB) which provides information in relation to:
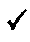 The demographic particulars of the holder;
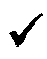 Document E111 which is necessary for medical coverage in the E.U.;
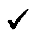 The medical history of the holder;
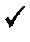 The medical insurance particulars of the holder;
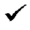 The particulars for medical exemption from work responsibilities for the holder, which with the specific access may be simply transmitted to his department or the employer through the Internet;
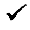 It provides a capacity for cooperation with specific medical packages for the enrichment of medical information as well as for the determination of medical appointments, etc.;

Due to the small size it provides the capacity to the user to always carry it with him, as with his credit card or identification or keys. Thus in the event of an accident or emergency admission into a Hospital, information shall be provided to the treating doctor, directly and without a loss of time, in relation to the prior medical condition of the patient, that is necessary and vital for the proper cover and treatment that shall be provided, e.g. blood group - allergies - heart condition - blood pressure - existing illnesses, etc. All of the information shall be presented directly and instantly on the Hospital computer screen and even on that for each treating doctor who shall examine the patient.

The data shall initially be entered onto the card by specific medical centres that are connected to the system and shall be updated at each visit that the patient makes to his doctor. It shall also be possible to enter onto the electronic card significant x-rays e.g. heart triple-x and breaks or deterioration to the bones which shall be instantly recalled in order that there may be proper and direct treatment both at the Hospital as well as at the private doctor, as they shall have the complete history for the patient. Thus medical faults due to a lack of information to the treating doctor shall disappear completely.

With the assistance of specific software needed destiny is provided for the reading and recording of particulars in the electronic book for the renewal of the medical history (the addition of a new examination or a medical action) or the alteration of the medical data for the holder (the highlight of some specific affliction or allergy that was discovered). Furthermore through the Internet there is the capacity for the authentication of the book by the holder and the dispatch of the particulars for examinations that were carried out by a particular doctor in order to render possible the financial compensation without the need to attend the offices of the insurer.

It also provides the capacity to the treating doctor through a specific access to support the **user doctor** in his communication with the centres that are supporting him and to remote medically cover him in the execution of his responsibilities.

The location of the EHB in emergency situations when the holder is not in possession of his senses by the ambulance or the hospital to which he is being admitted is rendered possible through a detection system that is attached to the EHB. The audible system receiver shall be installed into the EHB that shall be activated by the transmitter that shall be supplied by the ambulances and the hospitals, which shall be resistant and waterproof In the event where the holder desires it, he may also acquire the corresponding detection transmitter in order to easily locate the EHB when he has lost it or misplaced it, which is extremely useful for people with disabilities e.g. the blind.

To date these procedures are conducted through insurance books in a printed form, which in addition to everything else also have the two following disadvantages:
A. They are unable to store a large amount of information in relation to the medical history of the holder (such as x-rays or other types of photographic material); and
B. They compel the insured and the doctors to attend the offices of the insurer for the transaction of many tasks such as the authentication of the book, the authentication of examinations and financial compensation;

The storage medium for the particulars in the Electronic Health Book may be of two forms:
1. Electronic card for the storage of data that is connected to the USB port of a standard Personal Computer (USB flash memory stick);
2. A rewritable CD or DVD of a diameter of 8 cm or the dimensions of a credit card;

The necessary equipment for the provision of the complete service of the «Electronic Health Book» is:
1. A personal computer with a CD/DVD writing device in the event of the use of the second edition of the storage media;
2. Specific software that shall conduct the procedures for the reading / writing of the card and communication through the Internet with the insurer;

The electronic configuration of the card - book and the first recording of the personal particulars for the insured shall be conducted by specific software that assures the safe coding of the particulars in order to avoid the potential creation of forged books. The software for the reading of the book shall have the capacity for the recognition of its authenticity with the use of a suitable coding of the identity of the book holder. The book shall contain particulars in relation to the identity of the holder, personal medical information (blood group, allergies, etc), insurance particulars (coverage, authentications, etc) and the complete medical history with the capacity for the storage of text, images and electronic data for medical devices.

Every insured person shall be able to conduct whatever transaction with his insurer (such as the authentication of the book, the authentication of examinations and financial compensation) through the Internet, as long as he acquires the previously mentioned electronic apparatus (PC with access to the Internet and software). In the event where he does not desire the Internet transaction he may be able to submit his electronic book to his insurer and carry out there are whatever transaction.

In relation to every medical examination at a private doctor or a hospital that is connected to the insurer, the insured shall submit his electronic book which they will be able to read there and update it with the medical examinations carried out, through the electronic apparatus for the reading / recording. Furthermore the doctor or the hospital shall enter into the electronic book the examination or illness and shall be immediately informed in relation to the corresponding insurance coverage by the holder, in order to be able to conduct the charging of the necessary amount. In relation to the compensation for the doctor / hospital, they shall be able to attend the insurer either through the Internet or their offices by providing a coded electronic file that shall be created during the transaction with the insured and shall contain all the necessary information for the completion of the compensation.

## Claims

1. The Electronic Health Book that is comprised of the card for the store storage of information in an electronic form is **characterised by** the fact that it is suitably configured in order to contain the particulars for the identity of the holder, personal medical information, medical insurance particulars and the complete medical history with the capacity for the storage of text, images and electronic data relating to medical devices.

2. The arrangement of the electronic equipment for the reading and recording of the electronic card that is referred to above at claim 1 is **characterised by** the fact that it permits with the assistance of specific software, the following services:
A. The electronic configuration of the card in order to render possible the storage of the particulars that are referred to above at claim 1, in a cryptographic form;
B. The reading of the particulars of the card holder by the medical personnel for the conduct of treatment and suitable charging to the holder in accordance with his insurance cover;
C. The updating of the medical history for the patient by the medical personnel by a suitable recording on the card;
D. The updating of the insurance particulars of the card holder by the insurer (the capacity to also conducted this service through the Internet);
E. The generation of a cryptographic file that contains the necessary particulars for the card holder and the treatments in relation to the financial compensation to the doctor / hospital (the capacity to also transmit the file and complete the payment electronically through the Internet);

3. The Electronic Health Book in accordance with the claims at 1 and 2 is **characterised by** the fact that it acquires the capacity for the transmission of selected medical data for the holder by the use a Doctor to Medical centres that remote Medically support him, through a specific operation, in order to successfully and deal with the difficult Medical incidents.

4. The Electronic Health Book in accordance with the claims at 1, 2, and 3 is **characterised by** the fact that it has the capacity for its detection, through the transmitter - receiver system, to be easily detected. This is achieved through the insertion of an audible receiver signal, which is placed into the holder for the EHB.
